# EUROPEAN PATENT SPECIFICATION

(11) **EP 2 637 998 B1**
(45) Date of publication and mention of the grant of the patent: **22.08.2018**
(21) Application number: 11791113.1
(22) Date of filing: 09.11.2011
(51) Int. Cl.: C07C 67/54, C07C 51/44, C07C 51/573, C07C 53/08, C07C 53/12, C07C 69/18

(54) **PROCESS FOR REFINING CRUDE ACETYLS MIXTURE**
VERFAHREN ZUM RAFFINIEREN VON ROHEN ACETYLMISCHUNG
PROCÉDÉ POUR LE RAFFINAGE D'UN MÉLANGE D'ACÉTYLES BRUTS

(30) Priority: 12.11.2010 US 413224 P
(43) Date of publication of application: 18.09.2013
(73) Proprietor: Eastman Chemical Company, Kingsport, TN 37660 (US)
(72) Inventor: MAYFIELD, George, Geiger, Kingsport TN 37664 (US); LANE, Donald, Wayne, Kingsport TN 37663 (US); SCHISLA, Robert, Melvin, Kingsport TN 37664 (US); DYSON, Henry, Kingsport TN 37664 (US); BEWLEY, Jerry, Lee, Church Hill TN 37642 (US)
(74) Representative: Wibbelmann, Jobst
(86) International application number: PCT/US2011/059958
(87) International publication number: WO 2012/064832

(56) References cited:
- EP-A1- 0 087 870
- WO-A1-95/32938
- US-A- 5 292 948
- US-A- 5 554 790
- US-A1- 2009 299 092

## Description

### FIELD OF THE INVENTION

This invention pertains to a process for refining a crude acetyls mixture produced by carbonylation. More specifically, this invention pertains to a process for refining a crude acetyls mixture containing acetic anhydride, acetic acid, ethylidene diacetate and iodine-containing impurities to produce a mixture of acetic anhydride and acetic acid containing reduced levels of iodine-containing compounds and ethylidene diacetate impurities.

### BACKGROUND OF THE INVENTION

The preparation of acetic anhydride by contacting in the liquid phase a mixture containing methyl acetate and/or dimethyl ether and methyl iodide with carbon monoxide in the presence of a carbonylation catalyst has been reported extensively in the patent literature. See, for example, U.S. Patents 3,927,078; 4,046,807; 4,115,444; 4,252,741; 4,374,070; 4,430,273; 4,559,183; 5,003,104; 5,292,948 ; US2009/299092 and 5,922,911 and European Patents 8396, 87,869; and 87,870. The carbonylation process can be operated by feeding continuously the feedstock compound(s), methyl iodide, and catalyst or catalyst components, carbon monoxide, and solvents such as acetic acid, acetic anhydride, or mixtures of the two. Thus, the process mixture and the resulting product can contain a solution of the reactants as well as components of the feed, along with impurities such as ethylidene diacetate and iodine-containing compounds. Obtaining usable product requires separation of the undesired components from the reactant mixture. There is a continuing need to improve such separation processes.

### BRIEF SUMMARY OF THE INVENTION

The present invention provides a process for refining a crude acetyls mixture containing acetic anhydride, acetic acid, ethylidene diacetate and at least one iodine-containing compound, the process including the steps of:
(1) feeding the crude acetyls mixture to a first distillation column;
(2) removing from the first distillation column a first underflow stream containing acetic anhydride and ethylidene diacetate;
(3) removing from the first distillation column a first overhead vapor stream containing acetic acid and iodine-containing compounds;
(4) removing from the side of the upper section of the first distillation column a sidedraw stream containing acetic anhydride, acetic acid or a combination of the two; and
(5) feeding the first underflow stream of step (2) to a second distillation column and removing from the upper section of the second distillation column a second overhead vapor stream containing acetic anhydride and a second underflow stream containing ethylidene diacetate.

In some embodiments the first distillation column is operated at a column base column base pressure of 0.13 to 0.60 bar (100 to 450 torr) and a column head pressure of 0.05 to 0.33 bar (40 to 250 torr). In some embodiments the first distillation column is operated at a column base column base pressure of 0.27 to 0.53 bar (200 to 400 torr) and a column head pressure of 0.12 to 0.27 bar (90 to 200 torr). In some embodiments, the second distillation column is operated at a column base pressure of 0.13 to 0.60 bar (100 to 450 torr) and a column head pressure of 0.04 to 0.21 bar (30 to 160 torr). In some embodiments, the second distillation column is operated at a column base pressure of 0.13 to 0.53 bar (100 to 400 torr) and a column head pressure of 0.04 to 0.16 bar (30 to 120 torr).

The crude acetyls mixture fed to the first distillation column contains 5 to 80 weight percent acetic anhydride, 95 to 20 weight percent acetic acid, 0.2 to 2 weight percent ethylidene diacetate and 1 to 50 parts per million (ppm) iodine-containing compounds. In some embodiments, the concentration of iodine-containing compounds in the crude acetyl mixture is 3 to 10 ppm.

The acetic acid overhead stream of step (3) provides a means for the removal and recovery of a significant portion of the iodine-containing compounds fed to the first distillation column. Trace amounts of methyl acetate present in the crude acetyl mixture also may be recovered in the step (3) overhead stream. In some embodiments, the acetic acid and iodine-containing compounds obtained from step (3) may be returned to the carbonylation process, e.g., to a carbonylation feed vessel. In some embodiments, the acetic acid and iodine-containing compounds may be condensed and the condensed iodine-containing compounds and acetic acid fed to an esterification zone wherein acetic acid is reacted with methanol to produce methyl acetate. Thus, some embodiments of the present process include to step of:
(6) condensing the vapor stream of step (3) and feeding the condensed stream to an esterification zone wherein acetic acid is reacted with methanol to produce a methyl acetate product.

The recovery of iodine-containing compounds in the acetic acid stream of step (3) also provides an acetic acid/anhydride distillation product stream in the sidedraw removed in step (4) that contains reduced amounts of iodine-containing compounds. The reduced content of iodine-containing compounds of the step (4) sidedraw stream aids and enhances further purification procedures required to remove iodine-containing compounds since it is sometimes desirable to decrease the levels of iodine-containing compounds in acetic anhydride or acetic acid to very low levels, e.g., lower than 10 parts per billion. The sidedraw stream may optionally be further processed in downstream processes to further reduce levels of iodine-containing compounds.

The invention provides a means for reducing ethylidene diacetate content in crude acetyl mixtures at an early stage in the refining process, prior to the more fine distillation processes that separate acetic acid and acetic anhydride from each other and from other impurities. Due to its potential to form tar and other fouling components, ethylidene diacetate can create design or operational challenges in the finer distillation processes. Reducing or removing it early on provides more flexibility in designing downstream operations.

### DETAILED DESCRIPTION

As used throughout this application, the term "acid/anhydride" in reference to a composition or a component of a composition shall be used generically to describe a composition or a component of a composition that is acetic acid, acetic anhydride, or a combination of the two, and the term "acetic acid/anhydride" in reference to a composition or a component of a composition shall be used to describe a composition or a component of a composition that is acetic acid, acetic anhydride, or a combination of the two.

As used throughout this application, "iodine-containing compounds" means any compounds present in a crude acetyl mixture that include at least one iodine atom. Examples include C1 - C12 alkyl iodides (e.g. methyl iodide, ethyl iodide, propionyl iodide, butyl iodide, etc.), elemental iodine, acetyl iodide, iodomethyl acetate, iodoacetone, and iodoacetic acid. In some embodiments, the iodine-containing compound is one or more organic iodides. Some iodine-containing compounds have a sufficiently high volatility to be at least partially separated from ethylidene diacetate by boiling. Such iodine-containing compounds shall be referred to as "higher volatility" throughout this application, while those lacking such volatility will be referred to as "lower volatility." In some embodiments, the iodine-containing compound is one or more alkyl iodides. In some embodiments, the iodine-containing compound is methyl iodide.

It will be understood that while many parts of the inventive process are described as "steps," the invention does not preclude any or all of these steps from occurring simultaneously. All percentages of a component or composition refer to weight percentages (wt %) unless specifically stated otherwise.

### Crude Acetyls Mixture

The processes of the present invention will process crude acetyls mixtures. The crude acetyls mixture is a product stream containing acetic acid and/or acetic anhydride from a carbonylation process. The crude acetyl mixture may have previously been processed to remove or to recycle certain components, (for example through flash separation or distillation or combinations thereof) to remove unused feed materials, to remove catalyst materials for recycle or disposal, or to remove or to reduce the amount of iodine-containing compounds or other undesirable impurities.

The crude acetyls mixture fed to the first distillation column contains 95 to 100 weight percent acetic acid/anhydride, 0.2 to 2 weight percent ethylidene diacetate and 1 to 50 parts per million by weight (ppm) iodine-containing compounds. The relative ratio of acetic acid to acetic anhydride in the acid/anhydride component may be selected from a wide variety of values and ranges. Some examples include between 30:70 and 50:50, between 40:60 and 60:40, between 50:50 and 70:30, between 50:50 and 95:5, between 70:30 and 95:5, between 70:30 and 85:15, between 85:15 and 95:5, and so on. The crude acetyls mixture can also contain other compounds such as methyl acetate, catalyst components and high-boiling compounds such as process tar and compounds that are converted to tar. In some embodiments, the concentration of iodine-containing compounds in the crude acetyl mixture is between 0.5 and 50 ppm. In some embodiments, the concentration of iodine-containing compounds in the crude acetyl mixture is between 0.5 and 25 ppm. In some embodiments, the concentration of iodine-containing compounds in the crude acetyl mixture is between 0.5 and 10 ppm.

### First distillation column

The crude acetyls stream is fed to a first distillation column. The first distillation column separates the crude acetyls mixture into an underflow (2), an overhead (3) and a sidedraw (4). In some embodiments, the column has a reflux ratio below the sidedraw of between 0.5 and 1.5. The crude acetyls stream is fed at any effective point on the column, in some embodiments at a point between the top and the bottom, such as the approximate midpoint between the top and the bottom. In some embodiments it is fed between 25% and 50% of the length of the column up from the bottom.

In some embodiments the first distillation column is operated at a column base column base pressure of 0.19 to 0.60 bar (140 to 450 torr) and a column head pressure of 0.05 to 0.27 bar (40 to 200 torr). In some embodiments, the first distillation column is operated at a column base pressure of 0.27 to 0.53 bar (200 to 400 torr) and a column head pressure of 0.12 to 0.27 bar (90 to 200 torr). In some embodiments, the first distillation column is operated at a column base pressure of 0.27 to 0.47 bar (200 to 350 torr) and a column head pressure of 0.12 to 0.27 bar (90 to 200 torr). In some embodiments, the first distillation column is a column base pressure of 0.33 to 0.47 bar (250 to 350 torr); and a column head pressure of 10.17 to 0.19 bar (130 to 140 torr). In some embodiments, the first distillation column is operated at a column base temperature of 105 to 120°C and a column head temperature of 70 to 77°C. In some embodiments, the first distillation column is operated at a column base temperature of 105 to 120°C and a column head temperature of 70 to 75°C. By referring to a pressure or temperature as "column base," it is meant that value at the base or bottom of the column. By referring to a pressure or temperature as "column head," it is meant that value at the head or top of the column. The use of pressure below atmospheric (below 760 torr) allows operating the column at lower temperatures, which reduces the formation of tar which can foul the column internals and especially the reboiler that provides the heat for the operation of the column.

The sidedraw (4) is between the point at which crude acetyls mixture is fed to the column and the top reflux return, in some embodiments at the mid-point of the column. The sidedraw can contain the bulk of the product stream. In some embodiments, the sidedraw contains 60 to 95% of the crude acetyls stream. In some embodiments, the sidedraw contains 10 to 70% of the crude acetyls stream. This allows iodine-containing compounds to fractionate away from the acetyl product in the sidedraw. The optimal location of the sidedraw for a given set of design conditions (feed rate, feed composition, feed temperature and pressure, column height, column diameter, column pressure, packing or tray design, desired fractionation extent) may be determined by routine engineering calculations or with the aid of commercially available process simulation software, such as those available from Aspen Technology, Inc., Burlington, Massachusetts.

The distillation is effective to concentrate ethylidene diacetate in the step (2) underflow stream, although the invention does not require that all ethylidene diacetate go to the underflow. By "concentrate," it is meant that the weight percent of ethylidene diacetate present in the step (2) underflow stream is higher than that in the crude acetyls mixture fed to the first distillation column. In some embodiments, the step (2) underflow stream contains 70 to 99 weight percent acetic anhydride, and 1 to 30 weight percent ethylidene diacetate. In some embodiments, the underflow stream contains 85 to 97 weight percent acetic anhydride and 3 to 15 weight percent ethylidene diacetate. In some embodiments, the first distillation column is operated in a manner to result in at least 50% by weight of ethylidene diacetate from the crude acetyls mixture fed to the column to exit the column in the step (2) underflow. In various embodiments, the weight percent of ethylidene diacetate from the crude acetyls mixture that exits the column in the step (2) underflow is at least 60%, at least 75%, at least 85% or at least 90%. The step (2) underflow stream also may contain acetic acid. In some embodiments, the stream contains no acetic acid. In some embodiments, the stream contains 0-5% acetic acid. In some embodiments, the stream contains 0- 1% acetic acid. The step (2) underflow stream removed from the base of the first distillation column may also contain certain lower volatility iodine-containing compounds fed to the column. In some embodiments, the step (2) underflow stream contains 50 to 300 ppm lower volatility iodine-containing compounds. In some embodiments, the total amount of the step (2) underflow stream removed from the first distillation column is between 1 and 15 weight percent, in some embodiments between 2.5 and 10 weight percent based on the total weight of the crude acetyls mixture fed to the first distillation column.

The distillation is effective to concentrate higher volatility iodine-containing compounds and, if present in the crude acetyls mixture, methyl acetate in the step (3) overhead stream. By "concentrate," it is meant that the weight percent of higher volatility iodine-containing compounds present in the step (3) overhead stream is higher than that in the crude acetyls mixture fed to the first distillation column. In some embodiments, the overhead stream column contains 60 to 99 weight percent acetic acid, 0 to 40 weight percent acetic anhydride and 0.5 to 300 ppm higher volatility iodine-containing compounds. In some embodiments, the higher volatility iodine-containing compounds include methyl iodide. The ratio of acetic acid to acetic anhydride in the overhead is a function of the composition of the crude acetyls stream and the column parameters. Some examples that can be achieved include between 50:50 and 75:25, between 60:40 and 90:10, between 98:2 and 100% acetic acid, between 90:10 and 99:1, and between 90:10 and pure acetic acid. The step (3) overhead stream also may contain trace amounts of methyl acetate. In some embodiments, the step (3) stream contains up to 1 weight percent (wt %) methyl acetate. In some embodiments, the step (3) stream contains up to 0.25 wt % methyl acetate. In some embodiments, the total amount of the step (3) overhead stream removed is 2 to 40 weight percent based on the amount of the crude acetyls mixture fed to the first distillation column.

In some embodiments, the distillation in the first column is also effective to concentrate lower volatility iodine-containing compounds in the step (2) underflow stream. By "concentrate," it is meant that the weight percent of lower volatility iodine-containing compounds present in the step (2) underflow stream is higher than that in the crude acetyls mixture fed to the first distillation column.

The step (3) overhead stream may be handled in any useful manner. In some embodiments, the step (3) overhead stream is returned to the carbonylation process by feeding the stream to a carbonylation reactor feed tank. In some embodiments, the step (3) overhead stream is fed to a low boiler column, sometimes located upstream from the distillation columns of the present invention, wherein methyl iodide is recovered from the stream. In some embodiments of the invention, the step (3) overhead stream is fed to an esterification zone wherein acetic acid is reacted with methanol to form methyl acetate which may be used as a feedstock in the carbonylation process. US 4,435,595 describes one embodiment of an esterification zone for the production of methyl acetate from acetic acid and methanol.

In some embodiments, the step (4) sidedraw stream removed from the side of the upper section of the first distillation column contains concentrated acetic acid/anhydride, that is, the weight percent of acetic acid/anhydride present in the step (4) sidedraw stream is higher than that in the crude acetyls mixture fed to the first distillation column. The degree to which is weight-percent difference can be measured depends in part on the amount of contaminants that had been present in the crude acetyls mixture. In some embodiments, the step (4) sidedraw stream contains over 99% acid/anhydride, with 0 to 2 ppm iodine-containing compounds. As with the crude acetyls mixture, the relative ratio of acetic acid to acetic anhydride in the acid/anhydride component of the step (4) sidedraw stream may be selected from a wide variety of values and ranges. Some examples include between 30:70 and 50:50, between 40:60 and 60:40, between 50:50 and 70:30, between 50:50 and 95:5, between 70:30 and 95:5, between 70:30 and 85:15, between 80:20 and 90:10, between 85:15 and 95:5, between 90:10 and 100% acid, and so on. The result of concentrating higher volatility iodine-containing compounds in the underflow and concentrating lower volatility iodine-containing compounds in the overhead is to lower the concentration of iodine-containing compounds in the sidedraw. In some embodiments, the first distillation column is operated in a manner such that no more than 50% by weight of iodine-containing compounds in the crude acetyls mixture fed to the column will exit the column in the step (4) sidedraw. In various embodiments, the weight percent of iodine-containing compounds from the crude acetyls mixture that exit the column in the step (4) sidedraw is no more than 40%, no more than 25% or no more than 15%.

### Further processing of underflow from the first distillation column

The underflow from the first column is fed to a second distillation column. The step (2) underflow stream is fed at any effective point on the column, in some embodiments at a point between the top and the bottom, such as the approximate midpoint between the top and the bottom. The second column generates a second overhead stream containing acetic acid/anhydride and a second underflow stream containing acetic acid/acetic anhydride and ethylidene diacetate. The column acts to concentrate the ethylidene diacetate into the second underflow stream, although the invention does not require that the second overhead stream be free of ethylidene diacetate. By "concentrate," it is meant that the weight percent of ethylidene diacetate present in the second underflow stream is higher than that in the step (2) underflow stream fed to the second distillation column. In some embodiments, the second overhead stream contains from zero to 5 weight percent ethylidene diacetate. In some embodiments, the second overhead stream contains from zero to 2.5 weight percent ethylidene diacetate.

In some embodiments, the second distillation column is operated at a column base pressure of 0.13 to 0.60 bar (100 to 450 torr) and a column head pressure of 0.04 to 0.21 bar (30 to 160 torr). In some embodiments, the second distillation column is operated at a column base pressure of 0.13 to 0.19 bar (100 to 140 torr). and a column head pressure of 0.04 to 0.16 bar (30 to 120 torr). In some embodiments, the second distillation column is operated at a column base pressure of 0.13 to 0.33 bar (100 to 250 torr) and a column head pressure of 0.04 to 0.16 bar (30 to 120 torr). In some embodiments, the second distillation column is operated at a column base pressure of 0.13 to 0.27 bar (100 to 200 torr) and a column head pressure of 0.04 to 0.16 bar (30 to 120 torr). In some embodiments, the second distillation column is operated at a column base temperature of 85 to 140°C; a column head temperature of 70 to 90°C. In some embodiments, the second distillation column is operated at a column base temperature of 110 to 140°C; a column head temperature of 70 to 90°C.

In some embodiments, the second overhead from the second column constitutes 50 to 90 weight percent of the mixture fed to the second distillation column and contains at least 90 weight percent acetic acid/anhydride. In some embodiments, the second overhead stream contains at least 95 weight percent acetic acid/anhydride.

In some embodiments, the vapor product in the second overhead stream is recycled to the first distillation column, for example by condensing it and combining the condensed stream with the feed stream for the first distillation column or piping it to a feed tank that provides the feed to the first distillation column or to other upstream equipment.

In some embodiments, the second underflow from the second column contains 30 to 90 weight percent ethylidene diacetate, with acetic acid/anhydride as the primary impurity. In some embodiments, the second underflow contains 70 to 90 weight percent ethylidene diacetate. In some embodiments, the second distillation column is operated in a manner to result in at least 50% by weight of ethylidene diacetate from the step (2) underflow fed to the second distillation column to exit the second column in the second underflow. In various embodiments, the weight percent of ethylidene diacetate from the step (2) underflow that exits the second distillation column in the second underflow is at least 60%, at least 75%, at least 85% or at least 90%. Other impurities may include tar, other high-boiling components from the crude acetyls mixture and lower volatility iodine-containing compound. In some embodiments, the second underflow may be discarded or it may be treated to recover acetyl value or other valuable components.

### Further processing of sidedraw stream

The step (4) sidedraw stream may be processed further to obtain the desired compositions and purity. Such further processing may include, for example, further distillation under reduced pressure to separate acetic acid and acetic anhydride. However, the type of further processing involved is not critical, and any suitable or desired further processes or combination of processes may be used. Some examples include further distillations (*e.g*. removal of heavy fractions), hydrolysis of acetic anhydride to acetic acid, further processes to remove iodine-containing compounds, and combinations thereof.

The process described herein can be carried out in conventional distillation equipment such as columns that contain trays or packing materials, feed lines, take-off lines, heat sources such as reboilers to provide necessary process heat, and cooling sources such as condensers to provide necessary process cooling. The specific compositions of the acetic acid/anhydride streams removed from the first distillation column can be varied significantly depending on the raw acid/anhydride ratio fed into the refining system, the number of column stages, the amount of reflux used, and other commonly-implemented fractionation design parameters. The compositions of these streams depend on the particular design economic factors for a given installation and are not conditions for this particular invention.

The process of this invention is further illustrated by the following examples wherein flow rates are given in parts by weight and stream compositions are given in percentages by weight.

### EXAMPLES

### EXAMPLE 1

A mixture containing 35% acetic acid, 64.5% acetic anhydride, 0.5% ethylidene diacetate, 8 ppm iodine-containing compounds and traces of lower and higher boiling components was fed to approximately the mid-point of the side of a first distillation column at the rate of 225 parts per hour. The first distillation column contained 13 trays below the feed point and 3 packed beds (about 30 feet total) above the feed point, packed with slotted ring packing.

The column head temperature and column head pressure were maintained at approximately 77°C and 0.18 bar (135 torr), respectively. The column base temperature and column base pressure were maintained at approximately 117°C and 0.45 bar (335 torr). respectively. An overhead vapor stream containing 72% acetic acid, 28% acetic anhydride, 194 ppm iodine-containing compounds and trace amounts of methyl acetate was removed from the top of the first distillation column at the rate of 2 parts per hour. A sidedraw stream containing 35% acetic acid, 65% acetic anhydride, a trace of ethylidene diacetate and 1 ppm iodine-containing compounds was removed from the side of the first distillation column at the rate of 210 parts per hour. The sidedraw take-off point was located so that 75% of the refining stages were between the column feed point and the sidedraw takeoff point. An underflow stream containing 0.2% acetic acid, 91.7% acetic anhydride, 8% ethylidene diacetate, 95 ppm iodine-containing compounds and traces of higher boiling components was removed from the base of the first distillation column at the rate of 12.7 parts per hour. The column's reflux flow rate below the sidedraw was about 170 parts per hour.

The underflow from the first distillation column was fed to the mid-section of a second distillation column. The second column had 14 trays below the feed point and about 20 feet of 2" No. 2 HYPAK slotted ring packing above the feed point. The underflow products of both columns had heavy solids formed in the columns. The column head temperature and column head pressure were maintained at approximately 78°C and 0.10 bar (75 torr), respectively. The column base temperature and column base pressure were maintained at approximately 107°C and and 0.35 bar (260 torr). respectively. An overhead vapor stream containing 0.33% acetic acid, 99.42% acetic anhydride, 0.25% ethylidene diacetate and 2.1 ppm iodine-containing compounds was removed from the top of the second distillation column at the rate of 11.5 parts per hour. An underflow liquid stream containing 0.05% acetic acid, 10% acetic anhydride, 80% ethylidene diacetate, 989 ppm iodine-containing compounds and 10% high boilers including process tars was removed from the base of the second distillation column at the rate of 1.2 parts per hour. The second column used a reflux ratio of approximately 1.5:1.

### EXAMPLE 2:

A mixture that contained 83% acetic acid, 16.5% acetic anhydride, 0.6% ethylidene diacetate, 5.3 ppm iodine-containing compounds and traces of lower and higher boiling components was fed to approximately the midpoint of the side of a first distillation column at the rate of 100 parts per hour. The column used both packed and trayed internals. The top bed was 7.5 meters in height and was packed with 50 mm IMTP random packing or equivalent. The lower bed was 5 meters in height and was packed with 50 mm metal Pall rings or equivalent. The liquid sidedraw was located between the two beds, and was effected by a chimney tray or equivalent drawoff device. A portion of the sidedraw is recirculated to the column. The feed was located beneath the bottom bed. Below the feed, the column was trayed with 18 cross-flow fixed-valve or equivalent trays. The column head temperature and column head pressure were maintained at approximately 70°C and 0.20 bar (150 torr), respectively. The column base temperature and column base pressure were maintained at approximately and maintained at 108°C and 0.32 bar (241 torr), respectively. An underflow stream containing 1% acetic acid, 80% acetic anhydride, 18% ethylidene diacetate, 9.7 ppm iodine-containing compounds and traces of higher boiling components was removed from the base of the first distillation column at the rate of 2.9 parts per hour and fed to the mid-section of the second distillation column. An overhead condensed liquid stream containing 94% acetic acid, 6% acetic anhydride, 46 ppm iodine-containing compounds and trace amounts of methyl acetate was removed from the top of the first distillation column at the rate of 10.7 parts per hour. A sidedraw stream containing 83.2% acetic acid, 16.2% acetic anhydride, (ethylidene diacetate below detection limits) and 0.033 ppm iodine-containing compounds was removed from the side of the first distillation column at the rate of 87 parts per hour. The reflux rate below the sidedraw was about 51 parts.

In the second distillation column, the column head temperature and column head pressure were maintained at approximately 85°C and 0.11 bar (80 torr), respectively. The column base temperature and column base pressure were maintained at approximately and at 125°C and 0.25 bar (190 torr), respectively.
An overhead condensed liquid stream containing approximately 99% acetic anhydride, 1% acetic acid, (ethylidene diacetate and iodine-containing compound levels not available) was removed from the top of the second distillation column at the rate of 1.5 parts per hour. An underflow liquid stream containing 0.5% acetic acid, 7.5% acetic anhydride, 85% ethylidene diacetate, 45 ppm iodine-containing compounds, and about 7% high boilers including process tars was removed from the base of the second distillation column at the rate of 0.4 parts per hour.

## Claims

1. A process for refining a crude acetyls mixture comprising 5 to 80 weight percent acetic anhydride, 95 to 20 weight percent acetic acid, 0.2 to 2 weight percent ethylidene diacetate and 1 to 50 parts per million (ppm) iodine-containing compounds, the process comprising the steps of:
(1) feeding the crude acetyls mixture to a first distillation column;
(2) removing from the first distillation column a first underflow stream comprising acetic anhydride and ethylidene diacetate;
(3) removing from the first distillation column a first overhead vapor stream comprising acetic acid and iodine-containing compounds;
(4) removing from the side of the upper section of the first distillation column a sidedraw stream comprising acetic anhydride, acetic acid or a combination of the two; and
(5) feeding the first underflow stream of step (2) to a second distillation column and removing from the upper section of the second distillation column a second overhead vapor stream comprising acetic anhydride and a second underflow stream comprising ethylidene diacetate.

2. The process of claim 1, wherein the first distillation column is operated at a column base pressure of 0.19 to 0.60 bar (140 to 450 torr) and a column head pressure of 0.05 to 0.33 bar (40 to 250 torr).

3. The process of claim 1 or 2, wherein the second distillation column is operated at a column base pressure of 0.13 to 0.60 bar (100 to 450 torr) and a column head pressure of 0.04 to 0.21 bar (30 to 160 torr).

4. The process of any of claims 1-3, wherein the concentration of iodine-containing compounds in the crude acetyl mixture is 3 to 10 ppm.

5. The process of any of claims 1-4, wherein the process further comprises the step of:
(6) condensing the vapor stream of step (3) and feeding the condensed stream to an esterification zone wherein acetic acid is reacted with methanol to produce a methyl acetate product.

6. The process of any of claims 1-5, wherein the first underflow stream comprises iodine-containing compounds.

7. The process of any of claims 1-6, wherein the iodine-containing compounds comprise methyl iodide.

8. The process of any of Claims 1-7, wherein the first distillation column is operated in a manner to cause at least 75% by weight of the ethylidene diacetate from the crude acetyls mixture fed to the first distillation column to exit the first distillation column in the first underflow.

9. The process of any of Claims 1-7, wherein the first distillation column is operated in a manner to cause at least 90% by weight of the ethylidene diacetate from the crude acetyls mixture fed to the first distillation column to exit the first distillation column in the first underflow.

10. The process of any of Claims 1-9, wherein the process further comprises recycling the second overhead vapor stream to the first distillation column.

## Patentansprüche

1. Verfahren zur Raffinierung einer rohen Acetylmischung, umfassend 5 bis 80 Gew.-% Essigsäureanhydrid, 95 bis 20 Gew.-% Essigsäure, 0,2 bis 2 Gew.-% Ethylidendiacetat und 1 bis 50 Teile pro Million (ppm) lod-enthaltende Verbindungen, wobei das Verfahren die Schritte umfasst von:
(1) Einspeisen der rohen Acetylmischung in eine erste Destillationssäule;
(2) Entfernen von der ersten Destillationssäule eines ersten Unterflussstroms, umfassend Essigsäureanhydrid und Ethylidendiacetat;
(3) Entfernen von der ersten Destillationssäule eines ersten Überkopfdampfstroms, umfassend Essigsäure und lod-enthaltende Verbindungen;
(4) Entfernen von der Seite des oberen Bereichs der ersten Destillationssäule eines Seitenauslassstroms, umfassend Essigsäureanhydrid, Essigsäure oder eine Kombination der zwei; und
(5) Einspeisen des ersten Unterflussstroms aus Schritt (2) in eine zweite Destillationssäule und Entfernen von dem oberen Bereich der zweiten Destillationssäule eines zweiten Kopfüberdampfstroms, umfassend Essigsäureanhydrid und eines zweiten Unterflussstroms, umfassend Ethylidendiacetat.

2. Verfahren nach Anspruch 1, worin die erste Destillationssäule betrieben wird bei einem Säulenbasendruck von 0,19 bis 0,60 bar (140 bis 450 torr) und einem Säulenkopfdruck von 0,05 bis 0,33 bar (40 bis 250 torr).

3. Verfahren nach Anspruch 1 oder 2, worin die zweite Destillationssäule betrieben wird bei einem Säulenbasendruck von 0,13 bis 0,60 bar (100 bis 450 torr) und einem Säulenkopfdruck von 0,04 bis 0,21 bar (30 bis 160 torr).

4. Verfahren nach irgendeinem der Ansprüche 1-3, worin die Konzentration der lod-enthaltenden Verbindungen in der rohen Acetylmischung 3 bis 10 ppm ist.

5. Verfahren nach irgendeinem der Ansprüche 1-4, worin das Verfahren weiterhin den Schritt umfasst von:
(6) Kondensieren des Dampfstroms von Schritt (3) und Einspeisen des kondensierten Stroms in eine Veresterungszone, in der Essigsäure mit Methanol umgesetzt wird, um ein Methylacetat-Produkt zu erzeugen.

6. Verfahren nach irgendeinem der Ansprüche 1-5, worin der erste Unterflussstrom lod-enthaltende Verbindungen umfasst.

7. Verfahren nach irgendeinem der Ansprüche 1-6, worin die lod-enthaltenden Verbindungen Methyliodid umfassen.

8. Verfahren nach irgendeinem der Ansprüche 1-7, worin die erste Destillationssäule in einer Weise betrieben wird, dass mindestens 75 Gew.-% des Ethylidendiacetats von der rohen Acetylmischung, die in die erste Destillationssäule eingespeist wurde, die erste Destillationssäule in dem ersten Unterfluss verlässt.

9. Verfahren nach irgendeinem der Ansprüche 1-7, worin die erste Destillationssäule in einer Weise betrieben wird, dass mindestens 90 Gew.-% des Ethylidendiacetats von der rohen Acetylmischung, die in die erste Destillationssäule eingespeist wurde, die erste Destillationssäule in dem ersten Unterfluss verlässt.

10. Verfahren nach irgendeinem der Ansprüche 1-9, worin das Verfahren weiterhin das Rezyklieren des zweiten Kopfüberdampfstroms zu der ersten Destillationssäule umfasst.

## Revendications

1. Procédé de raffinage d'un mélange d'acétyles bruts comprenant 5 à 80 % en poids d'anhydride acétique, 95 à 20 % en poids d'acide acétique, 0,2 à 2 % en poids de diacétate d'éthylidène et 1 à 50 parties par million (ppm) de composés contenant de l'iode, le procédé comprenant les étapes de :
(1) alimentation du mélange d'acétyles bruts à une première colonne de distillation ;
(2) retrait à partir de la première colonne de distillation d'un premier courant de sousverse comprenant de l'anhydride acétique et du diacétate d'éthylidène ;
(3) retrait à partir de la première colonne de distillation d'un premier courant de vapeur de tête comprenant de l'acide acétique et des composés contenant de l'iode ;
(4) retrait à partir du côté de la section supérieure de la première colonne de distillation d'un courant latéral comprenant de l'anhydride acétique, de l'acide acétique ou une combinaison des deux ; et
(5) alimentation du premier courant de sousverse de l'étape (2) à une seconde colonne de distillation et retrait à partir de la section supérieure de la seconde colonne de distillation d'un second courant de vapeur de tête comprenant de l'anhydride acétique et d'un second courant de sousverse comprenant du diacétate d'éthy-lidène.

2. Procédé selon la revendication 1, dans lequel la première colonne de distillation fonctionne à une pression de base de colonne de 0,19 à 0,60 bar (140 à 450 torr) et une pression de tête de colonne de 0,05 à 0,33 bar (40 à 250 torr).

3. Procédé selon la revendication 1 ou 2, dans lequel la seconde colonne de distillation fonctionne à une pression de base de colonne de 0,13 à 0,60 bar (100 à 450 torr) et une pression de tête de colonne de 0,04 à 0,21 bar (30 à 160 torr).

4. Procédé selon l'une quelconque des revendications 1 à 3, dans lequel la concentration des composés contenant de l'iode dans le mélange acétyles bruts est de 3 à 10 ppm.

5. Procédé selon l'une quelconque des revendications 1 à 4, dans lequel le procédé comprend en outre l'étape de :
(6) condensation du courant de vapeur de l'étape (3) et alimentation du courant condensé à une zone d'estérification dans laquelle de l'acide acétique est fait réagir avec du méthanol pour produire un produit d'acétate de méthyle.

6. Procédé selon l'une quelconque des revendications 1 à 5, dans lequel le premier courant de sousverse comprend des composés contenant de l'iode.

7. Procédé selon l'une quelconque des revendications 1 à 6, dans lequel les composés contenant de l'iode comprennent de l'iodure de méthyle.

8. Procédé selon l'une quelconque des revendications 1 à 7, dans lequel la première colonne de distillation fonctionne de manière à amener au moins 75 % en poids du diacétate d'éthylidène du mélange d'acétyles bruts alimenté à la première colonne de distillation à sortir de la première colonne de distillation dans le premier sousverse.

9. Procédé selon l'une quelconque des revendications 1 à 7, dans lequel la première colonne de distillation fonctionne de manière à amener au moins 90 % en poids du diacétate d'éthylidène du mélange acétyles bruts alimenté à la première colonne de distillation à sortir de la première colonne de distillation dans le premier sousverse.

10. Procédé selon l'une quelconque des revendications 1 à 9, dans lequel le procédé comprend en outre le recyclage du second courant de vapeur de tête vers la première colonne de distillation.
